# EUROPÄISCHE PATENTANMELDUNG

(11) **EP 0 972 528 A2**
(43) Veröffentlichungstag der Anmeldung: **19.01.2000**
(21) Anmeldenummer: 99106013.8
(22) Anmeldetag: 25.03.1999
(51) Int. Cl.: A61K 51/10

(54) **Humantherapeutisch anwendbares Radioimmunkonjugat und Verfahren zu seiner Herstellung**

(30) Priorität: 27.03.1998 DE 19813687; 15.03.1999 DE 19911329
(71) Anmelder: Benes, Ivan Friedrich Dr. med. Dr. rer. nat. PhD., 8127 Forch (CH); Bosslet, Klaus, Dr., 13465 Berlin-Frohnau (DE)
(72) Erfinder: Benes, Ivan Friedrich Dr. med. Dr. rer. nat. PhD., 8127 Forch (CH); Bosslet, Klaus, Dr., 13465 Berlin-Frohnau (DE)
(74) Vertreter: Allgeier, Kurt

(57) **Zusammenfassung**

Die Erfindung betrifft eine Serie von Radioimmunkonjugaten, bei denen die α-Strahler oder β-Strahler mit oder ohne Verwendung von Komplexbildnern stabil an den Antikörper gekoppelt sind, wobei das Radioisotop kein Jod ist, bei denen die β-Strahler Yttrium-90, Rhenium-188, Rhenium-186, Kupfer-67 sowie Holmium-166 und Samarium-153 sein können und die α-Strahler Astatin-211 oder Bismuth-212 sein können und bei denen der MAk-Anteil des Konjugates von der Maus, dem Menschen oder anderen Säugetieren stammt sowie intakt, fragmentiert, humanisiert oder rekombinant manipuliert sein kann. Ausserdem betrifft die Erfindung Radioimmunkonjugate, bei denen der MAk an ein extrazelluläres Antigen, welches präferentiell auf Zellen des hämatopoetischen Systems vorkommt oder bei denen der MAk an ein Antigen auf der Oberfläche von Granulozyten oder Granulozyten-Vorläufern oder beiden Zellarten oder bei denen der MAk-Anteil des Konjugates an ein Epitop oder Epitope von CD 66 bindet, bzw. CD 66 a, b, c und e bindet.

## Beschreibung

Die Erfindung betrifft ein humantherapeutisch anwendbares Radioimmunkonjugat und ein Verfahren zu seiner Herstellung.

Die moderne Therapie maligner hämatopoetischer Erkrankungen besteht meist aus einer Kombination von zwei Behandlungen:
- Hochdosierte, differenzierte Chemotherapie oder Ganzkörperbestrahlung bzw. Kombination von beiden
- Knochenmark- bzw. Stammzelltransplantation

Durch die aggressive, hochdosierte, meist differenzierte Chemotherapie oder die Ganzkörperbestrahlung mit energiereichen, harten γ-Strahlen (z. Bsp. Hochvolttherapie mit einer ⁶⁰Co-Cobalt-Qelle) sowie durch die Kombination beider Therapiearten wird versucht, die aus dem Knochenmark stammenden bösartigen im Überschuss produzierten Zellen, sowie deren Vorlauferzellen am besten schon auf dem Niveau der gemeinsamen, pluripotenten Stammzellen des Knochenmarks semiquantitativ bzw. quantitativ abzutöten. Hierbei wird das blutbildende, granulozytenproduzierende sowie des thrombozytenbildende System im Knochenmark zerstört. Ohne weitere therapeutische Massnahmen würde diese starke Immunsuppression unweigerlich zum Tode des Patienten führen. Durch sofortige intravenöse Injektion von Knochenmarksuspensionen bzw. von allogenen Stammzellen oder auch wo immer möglich von autologem über purging gereinigtem Knochenmark wird jedoch das Überleben des Patienten gewährleistet. Die intravenös verabreichten Stamm- bzw. Knochenmarkzellen besiedeln die lymphoiden Organe wie Knochenmark und Milz. Durch Teilung, Differenzierung und Reifung entwickeln sie sich zu voll funktionsfähigen Blutbestandteilen (Erythrozyten, Granulozyten, Thrombozyten, Myelozyten, Monozyten), die bereits nach wenigen Wochen die durch die vorherige Chemotherapie und/oder Ganzkörperbestrahlung zerstörte Hämatopoese des Patienten wiederherstellen. Durch diese aufwendige Therapie ist in den letzten Jahren ein deutlich verbessertes Überleben bei einer Reihe von bösartigen hämatopoetischen Erkrankungen erzielt worden.

Als schwerwiegende Nebenwirkungen der hochdosierten Chemotherapie bzw. der Ganzkörperbestrahlung treten zusätzlich zu den durch die Zerstörung der Hämatopoese und der Immunsupression induzierten gravierenden Veränderungen weitere Schädigungen anderer lebenswichtiger Organe auf. Solche Organschädigungen manifestieren sich z. Bsp. durch Haarausfall, Übelkeit, Erbrechen und Unlust bei den Patienten.

Zusätzlich zu den mit hoher Frequenz auftretenden Nebenwirkungen wie Nierentoxizität (Miralbell et a. J Clin Oncol 1996 14 (2)579-585), Katarakten (Alevard T. Acta Oncol 1996, 35 (7) 137-140), Keimzell-Dysfunktion (Sarafoglou et al J Pediatr 1997, 130 (2) 210-216) tritt auch vereinzelt eine akute, die Atemwege bedrohende, nicht infektiöse Epiglottitis auf (Murray et al Bone Marrow Transplantation 1995, 15 (6) 997-998).

Da es sich bei den Patienten mit bösartigen hämatopoetischen Erkrankungen sehr häufig auch um Kinder handelt, besteht eine dringende medizinische Notwendigkeit, deren Behandlung sowohl bzgl. des Nebenwirkungsprofils als auch der Effizienz zu verbessern.

In den letzten 15 Jahren wurde eine Vielzahl von Versuchen unternommen, mittels unmarkierter monoklonaler Antikörper (MAk) bzw. radiomarkierter monoklonaler Antikörper (Radioimmunkonjugate) die Behandlung bösartiger Erkrankungen zu verbessern. Erst in den letzten fünf Jahren ist es allerdings gelungen, die klinische Wirksamkeit von unmarkierten zytotoxischen MAk zu belegen. So konnten Riethmüller et al. (Lancet 1994, 343, 1177-1183) in einer randomisierten klinischen Prüfung bei der Behandlung der "minimal residual disease" beim Kolonkarzinom mit einem zytotoxischen, gentechnisch nicht manipulierten MAk von der Maus eine statistisch abgesicherte Lebenszeit-Verlängerting belegen.

Bei der Behandlung von grösseren Tumormassen liess sich die klinische Wirksamkeit unmarkierter MAk bis jetzt noch nicht belegen, obwohl neben den MAk von der Maus auch humanisierte und gentechnisch manipulierte MAk sowie deren Fragmente benutzt wurden (Colnaghi et al. Current Opinion in Oncology 1993, 5, 1035-1042). Deshalb haben zahlreiche Autoren versucht, MAk (sowie deren gentechnische Varianten) unterschiedlicher Spezifität, Ursprungs und Grösse mit zytotoxischen Substanzen, wie z. Bsp. Zytostatika oder radioaktiven Isotopen zu markieren (Courtenay-Luck und Epenetos, Immunology 1990, 2 880-883). Hierbei wurden unterschiedliche Kopplungs- und Markierungsverfahren zur Bindung der toxischen Komponenten an den MAk verwendet.

Erst in den letzten Jahren ist es gelungen, für therapeutische Zwecke geeignete Radionuklide mit vorteilhaften Strahleneigenschaften, wie z. Bsp. Phosphor-32, Strontium-89, Yttrium-90, Samarium-153, Erbium-169, Ytterbium-175, Rhenium-188 (abgesehen von Jod, das bereits seit vielen Jahren an MAk gebunden werden kann, aber keine sehr günstigen Strahlungseigenschaften hat) mittels Hilfe von bifunktionellen Komplexbildnern stabil an MAk zu koppeln.

Radiochemische Methoden zur Markierung von MAk mit verschiedenen Radionukliden können in zwei Gruppen, die direkten sowie die indirekten Markierungsmethoden, unterteilt werden. Bei den direkten Methoden werden die inneren Disulfidbindungen (-S-S-) der Hinge-Region des MAk teilweise zu Sulfhydrilgruppen (-SH) reduziert. Dazu werden verschiedene Verbindungen mit reduzierenden Eigenschaften, wie die Derivate der Askorbinsäure (Hnatowich D et al. J Nucl Med 1994; 35: 127-134), bzw. Substanzen mit Sulfhydrilgruppen oder mit Zinn-II-Substanzen oder Komplexe (Mather S et al. J Nucl med 1990; 31: 692-697, Paik C et al J Nucl med Biol 1985; 12: 3-8, Rhodes B. J Nucl Med 1986; 27: 685-693, Thakur M. et al. Nucl Med Biol 1991; 18: 227-233, Schwarz A. et al J. Nucl Med 1986; 28: 721) verwendet. Versuche, Re-186 oder Re-188 stabil ohne Verwendung von Komplexbildnern an MAk zu koppeln, haben nach heutigem Stand der Erkenntnis (Visser al al J. Nucl Med 1993, 34, 1953-1963, siehe Seite 1962, Zeilen 34-38) zu Konjugaten geführt, die für die therapeutische Nutzung nicht ausreichend stabil sind (Griffiths et al. Cancer Res 1991, 51, 4594-4602, Su et al. J Nucl Med 1992, 33, 910).

Bei den indirekten Markierungsmethoden von MAk werden meist bifunktionelle Komplexbildner, wie Diamindithiol (Baidoo K. et al. Cancer Res. 1990; 50: 799-803), oder ein bifunktioneller Ester des NHS-BAT (Eisenhut M. et al. J Nucl Med 1991; 37: 362-370), oder Diamid-Dimercaptid (Kasina S. et al. J Nucl Med 1991: 32 1445-1451) bzw. DTPA Najafi A. et al. Int J Appl Radiat Isot 1984; 5; 554-557), oder ein neuer Komplexbildner, der auf der N₂S₄-Verbindung beruht (Najafi A. et al Nucl Med Biol 1991; 18: 179-185, Qu T. et al. Radiochim Acta 1993; 63:209-212) zur Konjugation des Radionuklids an den MAk verwendet. Alternative Methoden, die eine indirekte Bindung Konjugation) eines Radionuklids an den MAk ermöglichen, beruhen auf der Konjugation von Thiol-Gruppen an Aminosäuren (z. B. Lysin) im Proteinmolekül mit 2-Iminothiolan (Joiris E. et al. Nucl Med Biol 1991; 18: 353-356) oder mit den Gruppen von 1-imino-4-merkaptobutyl Verbindungen (Goedemans W. in Nicolin M. et al (eds.) Verona 1990; 595-603).

Besonders für die Komplexierung von Yttrium (vorzugsweise Y-90) geeignete Komplexbildner sind zum Beispiel DOTA (Denora et al. Anticancer Research 1997, 17, 1735-1744) oder 12 N4-Maleimid (tetraazocyclododecantextra-essig-säure) (Turner et al. Br. J. Cancer, 1994, 70: 35-41, und King et al. Cancer Res., 1994, 54: 6176-6185). Für die Komplexierung von Rhenium (vorzugsweise Re-186 oder Re-188) ist beispielsweise der MAG-3 Komplexbildner besonders geeignet (van Gog et al J Nucl Med 1996, 37, (2), 352-362). Allerdings haben diese Verfahren den Nachteil, dass eine Immunantwort gegen den Komplexbildneranteil des MAk-Komplexbildner-Konjugates gebildet werden kann.

Dieser Stand der verfügbaren therapeutischen Mittel zur Behandlung hochgradig maligner hämatopoetischer Erkrankungen ist wegen der massiv auftretenden Nebenwirkungen unbefriedigend. Aus diesen Gegebenheiten haben sich die Erfinder die Aufgabe gestellt, ein neuartiges Radioimmunkonjugat zu entwickeln und zur Verfügung zu stellen, durch dessen Anwendung die hochdosierte Chemotherapie oder Ganzkörperbestrahlung ganz oder zumindest in erheblichem Umfang ersetzt werden kann.

Nach der unveröffentlichten deutschten Patentanmeldung 198 13 687.0 wird ein neuartiges Radioimmunkonjugat vorgeschlagen (z. Bsp. mit Radionukliden konjugierte Immunglobuline) , bei dem α- oder β-Strahler (vorzugsweise Rhenium, Yttrium, Samarium u.a.) ohne Verwendung eines Komplexbildners an den MAk gekoppelt sind, sowie deren vorzugsweise Verwendung für die Behandlung hämatopoetischer Erkrankungen, aber auch sonstiger Tumore und Entzündungen vorgeschlagen. Diese Radioimmunkonjugate haben gegenüber den literaturbekannten mit Komplexbildnern erzeugten Radioimmunkonjugaten den Vorteil, dass sie keine Immunantwort gegen den Komplexbildner induzieren. Gegenüber den literaturbekannten Radioimmunkonjugaten, die ohne Komplexbildner hergestellt wurden, haben die erfindungsgemässen Radioimmunkonjugate den Vorteil, dass sie eine hohe Stabilität besitzen, die ihre therapeutische Verwendung möglich macht.

Desweiteren ermöglichen die bereits vorgeschlagenen Radioimmunkonjugate eine verbesserte Behandlung vor allem hämatopoetischer Erkrankungen dadurch, dass sie die hoch dosierte Chemotherapie und Ganzkörperbestrahlung ersetzen. Sowohl die Frequenz als auch die Stärke der Nebenwirkungen der Therapie mit diesen neuartigen Radioimmunkonjugaten ist niedriger als bei der Standard hoch dosierten Chemotherapie und Ganzkörperbestrahlung.

In Ergänzung dieser Vorschläge wurde gefunden, dass das erfindungsgemäss hergestellte Radioimmunkonjugat bei der Verwendung zur Mono-Therapie von akuten und chronischen myeloischen Leukämien zu sehr guten Behandlungserfolgen führt. Ferner wurde auch gefunden, dass das erfindungsgemäss hergestellte Radioummunkonjugat sowohl monotherapeutisch wie auch in Verbindung gezielter Chemotherapie oder mit Ganzkörperbestrahlung bei der Behandlung von akuten und chronischen lymphatischen Leukämien und Lymphonen zu guten Erfolgen führt.

Die neuen erfindungsgemässen Erkenntnisse erstrecken sich auch auf die erweiterte therapeutische Verwendbarkeit der Radioimmunkonjugate in Verbindung mit literaturbekannten Liganden mit Spezifität für lymphozitäre Antigene wie CD 19, CD 20, CD 22, HLL 2 und HLA DR 10β. Ein weiteres Anwendungsgebiet besteht in der therapeutischen Verwendbarkeit als bispezifische Antikörperradioimmunkonjugate nach Beispiel 14 mit Spezifität gegen granoluzitäre Antigene und lymphozitäre Antigene.

Nach zusätzlichen Erkenntnissen können die vorgenannten bispezifischen Antikörperradioimmunkonjugate nach Beispiel 14 mit Spezifität entsprechend Anlage 1, kombiniert mit Spezifitäten wie anti CD 19, anti CD 20, anti CD 22, anti HLA DR 10β oder HLL 2 therapeutisch verwendet werden.

Eine weitere therapeutische Verwendbarkeit der erfindungsgemässen Radioimmunkonjugate bei den vorgenannten granoluzytären und lymphozytären Antigenen ergibt die nachfolgende Anwendung von Komplexbildnern in Form von löslichen Salzen.

Als besonders vorteilhaft haben sich die Radioimmunkonjugate nach der Erfindung durch ihre weitere therapeutische Verwendbarkeit als Zweiphasen-Radioimmunkonjugate, bestehend aus einem biotinylierten MAk und α- oder β-Strahlern markierten Avidin zur Therapie von malignen hämatopoetischen Erkrankungen erwiesen, und darüber hinaus haben sich diese Zweiphasen-Radioimmunkonjugate, bestehend aus einem biotinylierten MAk mit α- oder β-Strahlern markierten aviden Bindemolekülen wie Streptavidin oder deren rekombinant, biochemisch oder peptidchemisch herstellbaren Fragmente zur Therapie von malignen, hämatopoetischen Erkrankungen erfolgreich einsetzen lassen.

Die Herstellung der erfindungsgemässen Radioimmunkonjugate entspricht den im Hauptpatent angegebenen Beispielen, wobei als Antikörper solche verwendet werden können, die mit Zeilen des hämatopoetischen Systems reagieren, wie z. Bsp. Granulozyten, Granulozyten-Vorläufer oder beiden Zelltypen. Besonders geeignet sind Antikörper, die mit CD 66 reagieren, wie z. Bsp. der in der Anlage 1 beschriebene MAk, welcher mit CD 66 a, b, c und e reagiert. Die Spezifität seiner V-Region ist durch die in Anlage 1 offenbarte cDNA Sequenz definiert.

Die hierzu genannten Beispiele sind folgende:

### Beispiel 1:

Ein MAk, wie der MAk, dessen V-Region in der Anlage 1 beschrieben ist, liegt gereinigt, stabilisiert und sterilfiltriert in Form eines klares Filtrates vor. Der MAk wird in einer sterilen Phosphat-Pufferlösung, z. Bsp. auf eine MAk Konzentration von 1 mg MAk/ml eingestellt. Diese Lösung wird mit Hilfe einer Ultrafiltrationseinheit auf ein Volumen ≤ 200 ml ankonzentriert und im Anschluss daran durch Diafiltration unter Zugabe von Phospat-Pufferlösung von Stabilisatoren etc. befreit und auf ein Volumen von 200 ml eingestellt. Der so gereinigte und ankonzentrierte MAk wird unter Zusatz von 2-Merkaptoäthanol im Molverhältnis 1000:1 (Merkaptoäthanol:MAk) unter leichtem Rühren für 30 Minuten bei Raumtemperatur teilweise reduktiv gespalten. Nach dieser Reaktionszeit wird der (Überschuss an 2-Merkaptoäthanol durch wiederholte Diafiltration mit je 200 ml einer mit sterilfiltriertem Stickstoff gesättigten Phospatpufferlösung diafiltriert und ständig auf den Restgehalt an 2-Merkaptoäthanol kontrolliert. Dabei muss ein Grenzwert von >1 µg/ml an Merkaptoäthanol erreicht werden. Die resultierende Antikörperkonzentration wurde bestimmt. Sie ist die Grundlage für die Berechnung der Einstellung der MAk-Endkonzentration (2.0 mg/ml) sowie der Stabilisatorzugabe (2 mg Sorbitol pro 1 mg MAk) in der abzufüllenden Lösung. Die klare, sterilfiltrierte, mit sterilfiltriertem Stickstoff gesättigte, fertige Abfüll-Lösung wird unverzügliche unter GMP-Bedingungen in gewaschene, entpyrogenisierte und sterilisierte Glasfläschchen, mit je einem Füllgewicht von 500 ± 10 mg, in je ein, mit flüssigem Stickstoff gekühltes Injektionsfläschchen, maschinell dosiert. Anschliessend werden Gefriertrocknungsstopfen auf die Fläschchen gesetzt und in einem Gefriertrockner unter automatischer Kontrolle eines Gefriertrocknungsprogrammes getrocknet, mit Stickstoff als Schutzgas begast und die Stopfen luftdicht verschlossen.

Die Markierung des lyophilisierten MAk mit einem Betastrahler, z. Bsp. mit dem Radionuklid Rhenium-188 in der chemischen Form als Natriumperrhenal, findet direkt in dem Fläschchen mit dem vorher reduzierten und lyophilisierten MAk statt. Zum lyophilisierten MAk wird zuerst eine Komplexlösung eines feinwirkenden Reduktionsmittels zugefügt, bestehend aus Tetranatrii1,1,3,3-propantetraphosphonas in Form eines Komplexes mit Zinn(II) (0,5 mg : 0,06 mg), das die Reduktion des MAk mild steuert. Erst nachher wird die Lösung des ¹⁸⁸Re-Natrium-perrhenates mit der gewünschten Aktivität im Bereich von 1,85-7,4 GBq (≅ 50-200 mCi), oder eines anderen β-Strahlers, hinzugefügt. Die ¹⁸⁸Re-Natriumperrhenat-Lösung wird vorher frisch aus einem ¹⁸⁸W-Wolfram/¹⁸⁸Re-Rhenium-Generators mit einer 0,9 %-igen Natriumchloridlösung eluiert.

### Beispiel 2:

Ein MAk, wie der MAk, dessen V-Region in der Anlage 1 beschrieben ist, liegt gereinigt, stabilisiert und sterilfiltriert in Form eines kleines Filtrates vor. Der MAk wird in einer sterilen Phosphat-Pufferlösung, z. Bsp. auf eine MAk Konzentration von 1 mg MAk/ml eingestellt. Diese Lösung wird mit Hilfe einer Ultrafiltrationseinheit auf ein Volumen ≤ 200 ml ankonzentriert und im Anschluss daran durch Diafiltration unter Zugabe von Phospat-Pufferlösung von Stabilisatoren etc. befreit und auf ein Volumen von 200 ml eingestellt. Die in einem Glasgefäss vorliegende gereinigte und ankonzentrierte MAk-Lösung wird unter Verwendung einer Schlauchpumpe, die über Silikon-Verbindungsschläuche mit einer flachen durchlaufenden Küvette (Volumen 3 ml) aus Quarzglas verbunden ist, rezirkuliert. Die im geschlossenen System zirkulierende MAk-Lösung wird mit strömendem sterilen Stickstoff fortlaufend begast. Im Bereich der Quarzglasküvette befindet sich ein geschlossenes, abgeschirmtes Quarzlampensystem, das mit 2 kompakten HQI-Halogen-Metalldampf-UV-Hochdruck-Quarzlampen (Leistung je 150 W, Typbezeichnung: HQI-TS 150 W/NDL, Hersteller Osram-Deutschland) ausgestattet ist. Die UV-Strahlenintensität in der Mitte der Quarzküvette beträgt 625 ± 10 µW·cm⁻². Die Spitzenleistung des UV-Spektrums dieser UV-Strahlers liegt bei Wellenlängen zwischen 250 - 260 nm, im Mittel bei 254 nm (80 %). Mit einem kleinen Anteil sind noch die Wellenlängen von 295 nm (1%), 365 nm (3%), 410 nm (2%), 470 nm (7%), 510 nm (1%) und 545 nm (6%). In diesem geschlossenen System wird die zirkulierende MAk-Lösung 30 Minuten der Wirkung von UV-Strahlen unter Bildung von H-Radikalen ausgesetzt. Bei dieser Reaktion werden die Disulfid-Brücken (-S-S-) der schweren Ketten der Immunglobuline zu Sulfhydril-Gruppen (-SH) reduziert, wodurch eine direkte Ankoppelung von Radionukliden ermöglicht wird.

Das weitere Verfahren, wie die Formulierung, Stabilisierung, aseptische Anfüllung, der Ablauf der pharmazeutischen Konfektionierung, Gefriertrocknung sowie Markierung der pharmazeutischen Injektionsform mit Radionukliden, besonders mit β-Strahlern, bevorzugt mit Yttrium-90, Strontium-89, Samarium-153, Rhenium-186, Rhenium-188 wird, wie im Beispiel 1 angegeben, durchgeführt.

### Beispiel 3:

Der im Beispiel 1 und 2 aufgeführte reduzierte MAk wird alternativ zu dem Tetranatrii-1,1,3,3-propantetraphosphonas-Zinn(II)-Komplex mit einem Komplex von Ethylendiamin-N,N,N',N'-tetrakis-[methylenphosphonas-Zinn (II)] als ein schonend wirkendes Reduktionsmittel, bei der Bindung und/oder Ankopplung eines Radionuklids bevorzugt einem β-Strahler, wie Yttrium-90, Strontium-89, Samarium-153, Rhenium-186, Rhenium-188, wie in Beispiel 1 angegeben, verwendet.

### Beispiel 4:

Der im Beispiel 1 und 2 aufgeführte reduzierte MAk wird alternativ zu dem Tetranatrii-1,1,3,3-propantetraphosphonas-Zinn(II)-Komplex mit einem Komplex von Diethylentriaminpenta [methylenphosphonas-Zinn (II)] als ein schonend wirkendes Reduktionsmittel bei der Bindung und/oder Ankopplung eines Radionuklids bevorzugt einem β-Strahler, wie Yttrium-90, Strontium-89, Samarium-153, Rhenium-186, Rhenium-188, wie im Beispiel 1 angegeben, verwendet.

### Beispiel 5:

Der im Beispiel 1 und 2 aufgeführte reduzierte MAk wird alternativ zu dem Tetranatrii-1,1,3,3-propantetra-phosphonas-Zinn(II)-Komplex mit einem Dizitrat-tri-Zinn(II)-Komplex als ein schonend wirkendes Reduktionsmittel, bei der Bindung und/oder Ankopplung eines Radionuklids bevorzugt einem β-Strahler, wie Yttrium-90, Strontium-89, Samarium-153, Rhenium-186, Rhenium-188, wie im Beispiel 1 angegeben, verwendet.

### Beispiel 6:

Ein MAk, wie der MAk, dessen V-Region in der Anlage 1 beschrieben ist, liegt gereinigt, stabilisiert und sterilfiltriert in Form eines kleines Filtrates vor. Der MAk wird in einer sterilen Phosphat-Pufferlösung, z. Bsp. auf eine MAk Konzentration von 1 mg MAk/ml eingestellt. Diese Lösung wird mit Hilfe einer Ultrafiltrationseinheit auf ein Volumen ≤ 200 ml ankonzentriert und im Anschluss daran durch Diafiltration unter Zugabe von Phospat-Pufferlösung von Stabilisatoren etc. befreit und auf ein Volumen von 200 ml eingestellt. Zu dem gereinigten und ankonzentrierten MAk wird, wie in Beispiel 1 angegeben, 2,3-Dihydroxy-2-cyclopenten-1-one, in einem Molverhältnis 500 : 1 hinzugefügt. Die Reaktionslösung lässt man unter leichtem Rühren und Stick-stoffbegasung bei Raumtemperatur über 20 Minuten reagieren. Dabei werden die Disulfidbrücken (-S-S-) der Hinge-Region des MAk teilweise durch Reduktion in reaktive Sulfhydrilgruppen (-SH) umgewandelt. Der Überschuss an 2,3-Di-hydroxy-2-cyclopenten-1-one wird nicht entfernt. Er dient als Stabilisator für den reduzierten MAk und beteiligt sich später bei der Ankopplung eines Radionuklids (z. Bsp. Yttrium-90, Rhenium-186, Rhenium-188 u.a.), an die Sulfhydrilgruppen des MAk. Die resultierende Antikörperkonzentration der MAk-Lösung wird durch Messung der Extinktion bestimmt, und dient als Grundlage zur Berechnung der Einstellung der MAk-Endkonzentration (2,0 mg/ml) sowie zur Stabilisatorzugabe (2,0 mg Sorbitol pro 1 mg MAk) in der abzufüllenden Lösung.

Das weitere Verfahren, wie die Formulierung, Stabilisierung, aseptische Abfüllung, Ablauf der pharmazeutischen Konfektionierung, Gefriertrocknung sowie die Markierung der pharmazeutischen Injektionsform mit Radionukliden, besonders mit β-Strahlern, bevorzugt mit Yttrium-90, Strontium-89, Samarium-153, Rhenium-186, Rhenium-188, wird, wie in Beispiel 1 angegeben, durchgeführt.

### Beispiel 7:

Ein MAk, wie der MAk, dessen V-Region in der Anlage 1 beschrieben ist, liegt gereinigt, stabilisiert und sterilfiltriert in Form eines kleines Filtrates vor. Der MAk wird in einer sterilen Phosphat-Pufferlösung, z. Bsp. auf eine MAk Konzentration von 1 mg MAk/ml eingestellt. Diese Lösung wird mit Hilfe einer Ultrafiltrationseinheit auf ein Volumen ≤ 200 ml ankonzentriert und im Anschluss daran durch Diafiltration unter Zugabe von Phospat-Pufferlösung von Stabilisatoren etc. befreit und auf ein Volumen von 200 ml eingestellt. Zu dem gereinigten und ankonzentrierten MAk wird, wie in Beispiel 1 angegeben, 5-Methyl-2,3-dihydroxy-2-cyclopenten-1-one, in einem Molverhältnis von 300 : 1 hinzugefügt. Die Reaktionslösung lässt man unter leichtem Rühren und Stickstoffbegasung bei Raumtemperatur über 20 Minuten reagieren. Dabei werden die Disulfidbrücken (-S-S-) der Hinge-Region des MAk teilweise durch Reduktion in reaktive Sulfhydrilgruppen (-SH) umgewandelt. Der Überschuss an 5-Methyl-2,3-dihydroxy-2-cyclopenten-1-one wird nicht entfernt. Er dient als Stabilisator für reduzierten MAk und beteiligt sich später bei der Ankopplung eines Radionuklids (z. Bsp. Yttrium-90, Rhenium-186, Rhenium-188 u.a.) an die Sulfhydrilgruppen des MAk. Die resultierende Antikörperkonzentration der MAk-Lösung wird durch Messung der Extinktion bestimmt, und dient als Grundlage zur Berechnung der Einstellung der MAk-Endkonzentration (2,0 mg/ml) sowie zur Stabilisatorzugabe (2,0 mg Sorbitol pro 1 mg MAk) in der abzufüllenden Lösung.

Das weitere Verfahren, wie die Formulierung, Stabilisierung, aseptische Abfüllung, Ablauf der pharmazeutischen Konfektionierung, Gefriertrocknung sowie die Markierung der pharmazeutischen Injektionsform mit Radionukliden, besonders mit β-Strahlern, bevorzugt mit Yttrium-90, Strontium-89, Samarium-153, Rhenium-186, Rhenium-188 wird, wie im Beispiel 1 angegeben, durchgeführt.

### Beispiel 8:

Der in den Beispielen 1, 2, 6 und 7 aufgeführte, jedoch vorher nicht reduzierte MAk, vorteilhafterweise der MAk mit Spezifität wie in Anlage 1 beschrieben, wird mit einem α-Amino-β-methyl-β-merkaptobutyric-Zinn(II)-Komplex, der sich wie ein Reduktionsmittel und gleichzeitig wie ein Komplexbildner verhält, zusammengetan. Dadurch wird eine Reduktion des MAk und eines Radionuklids mit einer gleichzeitigen Bindung eines Radionuklids an den MAk bewirkt. Bei dieser Reaktion wird ein Radionuklid wie z. Bsp. Yttrium-90, Strontium-89, Samarium-153, Rhenium-186, Rhenium-188, das in einer siebenwertigen Form vorliegt, wie z. Bsp. ¹⁸⁸Re-Natriumperrhenat (NaReO₄), verwendet.

### Beispiel 9:

Der in den Beispielen 1, 2, 6 und 7 aufgeführte, jedoch vorher nicht reduzierte MAk, vorteilhafterweise der MAk mit Spezifität wie in Anlage 1 beschrieben, wird mit einem Mercaptobernsteinsaure-Zinn(II)-Komplex, der sich wie ein Reduktionsmittel und gleichzeitig wie ein Komplexbildner verhält, zusammengetan. Dadurch wird eine Reduktion des MAk und eines Radionuklids mit einer gleichzeitigen Bindung eines Radionuklids an den MAk bewirkt. Bei dieser Reaktion wird ein Radionuklid, wie z. Bsp. Yttrium-90, Strontium-89, Samarium-153, Rhenium-186, Rhenium-188, das in einer siebenwertigen Form vorliegt, wie z. B. ¹⁸⁸Re-Natriumperrhenat (NaReO₄), verwendet.

Nach intravenöser Applikation der z. B. mit Rhenium 188 markierten Radioimmunkonjugate wird folgende relative Radioaktivitätsverteilung im Menschen beobachtet:

| | |
|---|---|
| Leber: | 8 % |
| Lunge: | 2 % |
| Milz: | 6 % |
| Nieren: | 4 % |
| Knochenmark: | 80 % |

Diese Radioaktivitätsverteilung belegt, dass das Re-188 Konjugat eines geeigneten gegen Granulozyten und Knochenmarks-Stammzellen gerichteten MAk, wie z.B. des MAk mit der in Anlage 1 beschriebenen V-Region, in der Lage ist, die Basalzellen sowie die lymphoiden Organe (vor allem das Knochenmark) sehr stark lokal in Einzeldosis und/oder fraktioniert durch einzelne Aktivitätsdosen zu bestrahlen. Demzufolge ist es möglich, mit Hilfe dieses Radioimmunkonjugates nach einer intravenösen Applikation therapeutischer Dosen eine komplette Eliminierung des gesamten hämatopoetischen Systems zu erzielen und nicht hämapoetische Organe zu schonen.

Eine vergleichbare Radioaktivitätsverteilung wurde mit einem Radioimmunkonjugat erzielt, welches aus einem MAk besteht, der die in Anlage 1 definierte V-Region besitzt, und bei dem das Radionuklid über einen bifunktionellen Komplexbildner (Isothiocyanatobenzyl-DTPA) (Camera et al., Eur. J. Nucl. Med., 21, 640-646, 1994) gekoppelt ist. Solche Radioimmunkonjugate, bei denen das Nuklid über literaturbekannte Komplexbildner an den MAk gebunden ist, können ebenfalls verwendet werden, um die in Beispiel 9 oben erreichte vorteilhafte Organverteilung (hohe Aktivität im Knochenmark) zu erzielen.
Demzufolge erstreckt sich die erfindungsgemässe Verwendung von Radioimmunkonjugaten auf alle Konjugate, die nach systemischer Applikation eine präferentielle lokale Bestrahlung des hämatopoetischen Systems bewirken.

### Beispiel 10:

In den Beispielen 1 - 9 wurden die erfindungsgemässen MAk unter zu Hilfenahme von Komplexbildnern und Reduktionsmitteln mit dem β-Strahler verknüpft.

Besonders stabile Komplexe aus Y-90 und MAk wurden unter der Verwendung folgender Stoffe und Methode erreicht:

Der nicht reduzierte MAk wurde mit Isothiocyanat Zitronensäure oder Isothiocyanat-Benzyl-Zitronensäure oder Isothiocyanat-Acetyl-Zitronensäure nach literaturbekannten Bedingungen (Meares et al: Analytical Biochemistry, 142, 98-78, 1984) umgesetzt.

Das entstandene MAk-Zitronensäurekonjugat wird mittels PD 10 Gelpermeationschromatographie (Pharmacia Biotech AB, Upsala Schweden) von den niedermolekularen Kontaminanten nach literaturbekannten Verfahren getrennt (Safavy et al. Bioconjugate Chem. 10, 18-23, 1999 Seite 20).

Von dem so gereinigten MAk-Zitronensäurekonjugat werden z. Bsp 2 mg mit 100 mCi trägerfreiem Y-90 Chlorid gemischt (Aktivitätskonzentration: 100 mCi/0.5 ml). Nach 10-minütiger Inkubation bei Raumtemperatur erfolgt eine quantitative Bindung des Nuklids an das MAk-Konjugat.

25-100 mCi des erzeugten Radioimmunkonjugates werden mit 2 ml physiologischer Kochsalzlösung verdünnt und innerhalb von 5 Minuten i.v. an Patienten verabreicht, die für eine Kochenmarkskonditionierung vorgesehen sind.

Die verabreichte Aktivität wird innerhalb von 20 Minuten zu 80 % im Knochenmark, zu 8 % in der Leber, zu 6 % in der Milz, zu 2 % in der Lunge und 4 % in den Nieren wiedergefunden. Die im Körper an Granulozyten gebundene und im Blut zirkulierende Aktivität wird mit einer Eliminierungsrate von 4 %/24 Stunden ausgeschieden.
Die sich im Knochenmark befindende Aktivität verbleibt bis zum Zerfall des Nuklids im Knochenmark.
Diese lokale Bestrahlung des Knochenmarks führt zu einer kompletten Radioeliminierung der pluritpotenten Stammzellen des Knochenmarkes.

Im Gegensatz zu literaturbekannten MAk-DTPA oder MAk-DOTA Chelaten (die sich als immunogener Hapten Carrier Komplex verhalten können), induzieren die hier beschriebenen MAk-Zitronensäure-Komplexe keine Antikörperantwort gegen das Hapten.

Im Gegensatz zu dem obenerwähnten nicht reduzierten MAk, besteht die Möglichkeit unter Verwendung von Thiomethyl-acetyl-Citronensäure (wird genau beschrieben), auch den in den Beispielen 1 - 8 beschriebenen reduzierten MAk zu verwenden.

Anstelle von Merkaptoäthanol zur Reduktion des MAk können in den obengenannten Beispielen auch andere Reduktionsmittel verwendet werden, vorzugsweise bestimmte Phosphine (Literaturzitat).

### Beispiel 11

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert.

12 Patienten mit akuter bzw. chronischer myeloischer Leukämie (AML, CML) wurden in fraktionierten Dosen in einwöchigen Abständen Aktivitäten von 100 mCi pro Applikation intravenös verabreicht. Die Aufgabe dieser Behandlung ist es, nicht nur die leukämische Blastenpopulation im Knochenmark und in den anderen Organen sondern hauptsächlich auch die pluripotenten Blutstammzellen vollständig zu zerstören und dadurch eine gesunde Repopulation des Knochenmarks zu ermöglichen. Nach Durchführung geeigneter zytologischer Knochenmarks- und Blutbilduntersuchungen wurden die Patienten mit autologem bzw. allogenem Knochenmark bzw. Blutstammzellen entsprechend dem Stand der Technik transplantiert. Die so mit Radioimmunkonjugat-Monotherapie behandelten Patienten akzeptieren die Knochenmarkstransplantation in einem wesentlich höheren Prozentsatz (12 von 12) als Patienten, die mit der zur Zeit üblichen Chemotherapie mit toxischen Zytostatika als Standardtherapie behandelt wurden (maximal 60%). Desweiteren wurde der Prozentsatz an Rezidiven unter Radioimmunkonjugat-Monotherapie im Vergleich zur Zytostatikabehandlung deutlich reduziert. Die Ursache für diese überlegenen Effekte ist die spezifischere und effizientere Zerstörung der Blutstammzellen im Knochenmark, aus denen die akuten und myeloischen Leukämien hervorgehen, durch die Strahlenenergie.
Erklärbar sind diese therapeutischen Effekte durch die im folgenden näher beschriebene Dosisverteilung im Patienten:

| Organ | Gesamtdosis (Gray) |
|---|---|
| Knochenmark | 15-20 |
| Leber | 3-5 |
| Nieren | 5-7 |

Im Zielorgan für diese Therapie, dem Knochenmark, einem strahlenempfindlichen Organ, reichert sich ungefähr 80 % der verabreichten Dosis an, Leber und Niere, relativ strahlenresistente Organe, werden nur unwesentlich mit Strahlen belastet.
Im Gegensatz hierzu verteilen sich die hoch toxisch wirkenden Zytostatika unspezifisch im ganzen Körper, so dass nicht nur das Zielorgan sondern auch die lebenswichtigen Organe wie die Leber, Nieren, Lungen in starke Mitleidenschaft gezogen werden.

### Beispiel 12:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radioaktiv markiert. Patienten mit akuter (ALL) bzw. chronischer lymphatischer Leukämie (CLL) (6 x ALL, 8 x CLL) wurden in fraktionierten Dosen in einwöchigen Abständen Aktivitäten von 100 mCi pro
Applikation intravenös verabreicht (100 mCi/Woche x 2), In der dritten Woche wurden die Patienten mit Ganzkörperbestrahlung (12 Gray) und Busulfan behandelt. Nach Durchführung geeigneter zytologischer Knochenmarks- und Blutbilduntersuchungen wurden die Patienten mit autologem bzw. mehr mit allogenem Knochenmark bzw. Stammzellen entsprechend dem Stand der Technik transplantiert. Die so mit Kombinationstherapie bestehend aus Radioimmuntherapie, Ganzkörperbestrahlung und Chemotherapie behandelten Patienten gingen alle (14 aus 14) in komplette Remission. Desweiteren wurde das rezidivfreie Intervall von 6 Monaten auf > 9 Monate verlängert.

Im Gegensatz zur Behandlung der AML und CML, die mit Radioimmun-Monotherapie behandelt werden konnte, musste bei der ALL und CLL neben dem Knochenmark aufgrund der verschiedenen Krankheitsverteilung, noch das lymphatische System (Lymphknoten) therapiert werden. Dies wird durch die zusätzliche Ganzkörperbestrahlung und Chemotherapie erreicht.

### Beispiel 13:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert.
Patienten mit akuter bzw. chronischer lymphatischer Leukämie (13 x ALL, 12 x CLL) wurden in fraktionierten Dosen in einwöchigen Abständen Aktivitäten von 50 mCi pro Applikation intravenös verabreicht (50 mCi/Woche x 2). Gleichzeitig wurde den Patienten anti CD 20 MAk (RituxiMab) injiziert, der nach den
Beispielen 1 oder 2 oder 3 oder 4 bis 10 mit alpha- oder beta-Strahlern radiomarkiert wurde. Nach Durchführung geeigneter zytologischer Knochenmarks- und Blutbilduntersuchungen wurden die Patienten mit autologem bzw. allogenem Knochenmark bzw. Stammzellen entsprechend dem Stand der Technik transplantiert.
80 % der behandelten Patienten wurden in komplette Remission überführt (20 aus 25) Eine solch hohe Remissionsrate wurde nur deshalb erreicht, weil durch die Kombinationsradiotherapie mit anti B-Zell- und anti Granulozyten MAk sowohl eine effiziente Lympknoten als auch Knochenmarksbestrahlung ermöglicht wurde.

### Beispiel 14:

Ein bispezifischer MAk, selektiv für Lymphozyten, Granulozyten oder Granulozyten-Vorläufern, der entsprechend EP 0 517 024 B1 hergestellt wurde, wurde nach der in den Beispielen 1 oder 2 oder 3 oder 4 bis 10 beschriebenen Methodik mit alpha- oder beta-Strahlern radiomarkiert. Patienten mit akuter bzw. chronischer lymphatischer Leukämie (8 x ALL, 12 x CLL) wurden in fraktionierten Dosen in einwöchigen Abständen Aktivitäten von 100 mCi pro Applikation intravenös verabreicht (100 mCi/Woche x 2). Nach Durchführung, geeigneter zytologischer Knochenmarks- und Blutbilduntersuchungen wurden die Patienten mit autologem bzw. allogenem Knochenmark bzw. Stammzellen entsprechend dem Stand der Technik transplantiert.

90 % der behandelten Patienten wurden in komplette Remission überführt (18 aus 20). Eine solch hohe Remissionsrate wurde nur deshalb erreicht, weil durch die Bispezfität des Konstruktes sowohl eine effiziente Lymphknoten- als auch Knochenmarksbestrahlung ermöglicht wurde.

### Beispiel 15:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert.

3 Patienten mit akuter bzw. chronischer myeloischer Leukämie wurde in fraktionierten Dosen in einwöchigen Abständen eine Aktivität von 100 mCi pro Applikation intravenös verabreicht.
1 - 4 Stunden nach Injektion des Radioimmunkonjugates wird dem Patienten eine toxikologisch unbedenkliche Dosis (20 mg / kg KG des Patienten) an z. B. Dinatrium-Calcium-EDTA (z. B. Edtacal) oder mit Vorteil Calcium-DTPA (z. B. Ditipentat) oder Zink-DTPA oder Calciummerkapto-Bernsteinsäure oder ein anderer toxikologisch unbedenklicher Komplexbildner intravenös appliziert. Durch diese Verabreichung wird das vom Radioimmunkonjugat freigesetzte Nuklid aus allen extrazellulären Kompartimenten entfernt und über die Harnwege in kürzester Zeit ausgeschieden. Dadurch wird die unspezifische Strahlendosis aus dem Normalgewebe des Körpers weiter reduziert. Anschliessend werden geeignete zytologische Knochenmarks- und Blutbilduntersuchungen durchgeführt. Die weitere Therapie erfolgt wie in Beispiel 11 beschrieben.

### Beispiel 16:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlen radiomarkiert (Radioimmunkonjugat). 4 Patienten mit malignem Pemphigus wurde in fraktionierten Dosen in einwöchigen Abständen ein Radioimmunkonjugat mit einer Aktivität von 100 mCi pro Applikation intravenös verabreicht. 1 - 4 Stunden nach Injektion des Radioimmunkonjugates wird dem Patienten eine toxikologisch unbedenkliche Dosis (20 mg/kg KG des Patienten) an z. Bsp. Dinatrium-Calcium-EDTA (z. B. Edtacal) oder vorteilhafterweise Calcium-DTPA (z. B. Ditipentat) oder Zink-DTPA oder Calciummerkapto-Bernsteinsäure oder ein anderer toxikologisch unbedenklicher Komplexbild-ner intravenös appliziert. Durch diese Verabreichung wird das vom Radioimmunkonjugat freigesetzte Nuklid aus allen extrazellulären Kompartimenten entfernt und über die Harnwege in kürzester Zeit ausgeschieden. Dadurch wird die unspezifische Strahlendosis aus dem Normalgewebe des Körpers weiter reduziert. Die so mit Radioimmunkonjugat-Monotherapie behandelten Patienten akzeptierten alle die Knochenmarktransplantation. Nach einigen Wochen waren bei allen Patienten die Symptome des malignen Pemphigus verschwunden.

### Beispiel 17:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert (Radioimmunkonjugat). 2 Patientinnen mit fortgeschrittenem metastasierendem Mammakarzinom und ossären Metastasen wurde in fraktionierten Dosen in einwöchigen Abständen eine Radioimmunkonjugat-Aktivität von 100 mCi pro Applikation intravenös verabreicht (100 mCi/Woche x 2). Danach erhielten die Patienten eine Hochdosis Chemotherapie gefolgt von einer Knochenmarktransplantation.
Beide Patienten akzeptierten das Knochenmark. Die ossären Metastasen konnten quantitativ entfernt werden; beide Patienten wurden in eine komplette Remission überführt.

### Beispiel 18:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert (Radioimmunkonjugat). 3 Patienten mit hormonunabhängigem metastasierendem Prostatakarzinom und ossären Metastasen wurde in fraktiopierten Dosen in einwöchigen Abständen eine Radioimmunkonjugat-Aktivität von 100 mCi pro Applikation intravenös verabreicht (100 mCi/Woche x 2). Danach erhielten die Patienten eine Knochenmarktransplantation.
Alle 3 Patienten akzeptierten das Knochenmark. Die ossären Metastasen waren quantitativ entfernt.

### Beispiel 19:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert (Radioimmunkonjugat). 2 Patienten mit fortgeschrittenem non-Hodgkin Lymphom mit Ausdehnung ins Knochenmark wurde in fraktionierten Dosen in einwöchigen Abständen eine Radioimmunkonjugat-Aktivität von 100 mCi pro Applikation intravenös verabreicht (100 mCi/Woche x 2) - Danach erhielten die Patienten eine Hochdosis Chemotherapie gefolgt von einer Knochenmarktransplantation. Beide Patienten akzeptierten das Knochenmark. Nach der Behandlung konnten keine Lymphom-Metastasen im Knochenmark mehr nachgewiesen werden.

### Beispiel 20:

Ein MAk, wie der MAk, dessen V-Region in der Anlage 1 beschrieben ist, wurde wie in den Beispielen 1 oder 2 oder 3 oder 4 bis 10 beschrieben mit alpha- oder beta-Strahlern radiomarkiert (Radioimmunkonjugat). Je 1 Patient mit Knochenmarksmetastasen eines Lungenkarzinoms, Mammakarzinoms, Prostatakarzinoms, Pankreaskarzinoms, Magenkarzinoms, Leberkarzinoms, Nierenkarzinoms, Colonkarzinoms, Rektumkarzinoms, Hodentumors, Ovarialkarzinoms, Melanoms, Lymphoms, Mesothelioms, Kaposisarkoms, Hämangioms, Sarkoms, Osteosarkoms, Blasenkarzinoms und Hals-, Nasen-, Ohren-Karzinoms wurde mit fraktionierten Dosen in einwöchigen Abständen mit einer Radioimmunkonjugat-Aktivitat von 100 mCi pro Applikation intravenös behandelt (100 mCi/Woche x 2).

Nach Knochenmarktransplantation konnten bei keinem der Patienten Metastasen im Knochenmark nachgewiesen werden.

### Beispiel 21:

Nach dem aktuellen Stand der Knochenmarktransplantation werden die für die Transplantation vorgesehenen Patienten vor dem Erhalt des neuen Knochenmarks zum Beispiel mit Ganzkörperbestrahlung behandelt, um eine ausreichende Konditionierung des Knochenmarkes der Patienten zu erreichen. Da die Ganzkörperbestrahlung nicht nur das Knochenmark sondern auch die Normalgewebe bestrahlt, ist diese Behandlung mit starken Nebenwirkungen verbunden.

Demzufolge wurde 3 für die Knochenmarktransplantation vorgesehenen Patienten 100 mCi des Radioimmunkonjugates aus Beispiel 20 in einwöchigen Abständen intravenös appliziert (100 mCi/Woche x 2).

Anschliessend erhielten die Patienten allogenes Knochenmark transplantiert. Alle Patienten haben das Knochenmark akzeptiert. Die beobachteten Nebenwirkungen waren auf Übelkeit, leichtes Erbrechen, Appetitlosigkeit bei 50 % der Patienten, WHO Grad I/II beschränkt. Es wurden weder Schleimhautschäden noch vaskuläre Komplikationen beobachtet.

### Beispiel 22:

Ein MAk, wie der MAk, dessen V-Region in der Anlage 1 beschrieben ist, wurde nach literaturbekannten Methoden (siehe Anlage) mit Biotin markiert. Das niedermolekulare nicht an den MAk gebundene Biotin wurde über eine Sephadex G-20 Säule abgetrennt.
Je 2 Patienten mit AML bzw. CML wurden je 100 mg MAk-Biotinkonjugat intravenös verabreicht. Nach einem Zeitraum von 5 bzw. 10 Tagen erhielten die Patienten eine Injektion von je 100 mCi Avidin, welches entsprechend der in den Beispielen 1 - 8 für MAk beschriebenen Methoden mit α oder β-Strahlern markiert war. Mit Hilfe dieser Zweiphasenmethode war es möglich eine noch höhere Strahlendosis als bei der Therapie mit Radioimmunkonjugaten auf dem Zielgewebe, dem leukämischen Knochenmark, abzulagern.
Alle 4 Patienten wurden mit dieser Behandlungsmethode in eine komplette Remission überführt.
Der Vorteil dieser Behandlungsmethode gegenüber der Behandlung mit Radioimmunkonjugat Einphasentherapie ist, dass durch die Applikation hoher Dosen des MAk-Biotinkonjugates eine Sättigung der auf den Zellen des hämatopoetischen Systems vorhandenen Bindungsstellen möglich ist. Die nachfolgende Injektion mit radiomarkiertem Avidin führt auf Grund der hohen Avidität des Avidins zum MAk-Biotinkonjugat zu einer sehr effizienten Lokalisation des Avidins am MAk-Biotinkonjugat, d. h. im leukämischen Knochenmark. Mit Hilfe dieser Technik wird in vivo ein für die Therapie vorteilhaftes Zweiphasen-Radioimmunkonjugat erzeugt, welches eine noch höhere Strahlendosis am Zielgewebe appliziert als die in dieser Erfindung beschriebenen Einphasen-Radioimmunkonjugate (Radioimmunkonjugate). Anstelle des Avidins können auch andere avide Bindemoleküle wie z. Bsp. Streptavidin oder Fragmente solcher aviden Bindemoleküle die rekombinant, biochemisch oder peptidchemisch herstellbar sind, verwendet werden.

Anlage 1:

Sequenzprotokoll:
Art der Sequenz: | Nukleotid mit entsprechendem Protein
Sequenzlänge: | 357 Basenpaare
Strangform: | Einzelstrang
Topologie: | linear
Art des Moleküls: | cDNA zu mRNA
Ursprünglicher Herkunftsorganismus: | Maus

Merkmale:
cDNA Sequenz der Codons für die Aminosäuren 1-119 des VH Exons des schwere Ketten-
Gens, das für die schwere Kette des MAk kodiert und die dazugehörige Aminosäuresequenz.
Die Aminosäuren sind nach Wu und Kabat nummeriert.

VH
1 5 10 15
CAG GTC CAA CTG CAG GAG TCT GGA GGA GGC TTG GTA CAG CCT GGG GGT
Gln Val Gln Leu Gln Glu Ser Gly Gly Gly Leu Val Gln Pro Gly Gly

20 25 30
TCT CTG AGA CTC TCC TGC GCA ACT TCT GGG TTC AGT GAT TAC TAC ATG
Ser Leu Arg Leu Ser Cys Ala Thr Ser Gly Phe Ser Asp Tyr Tyr Met

35 40 45
AAC TGG GTC CGC CAG CCT CCA GGA AAA GCA CTT GAG TGG TTG GGT TTT
Asn Trp Val Arg Gln Pro Pro Gly Lys Ala Leu Glu Trp Leu Gly Phe

50 55 60
ATT TCA AAC AAA CCT AAT GGT CAC ACA ACA GAG TAC AGT GCA TCT GTG
Ile Ser Asn Lys Pro Asn Gly His Thr Thr Glu Tyr Ser Ala Ser Val

65 70 75 80
AAG GGT CGG TTC ACC ATC TCC AGA GAT AAT TCC CAA AGC ATC CTC TAT
Lys Gly Arg Phe Thr Ile Ser Arg Asp Asn Ser Gln Ser Ile Leu Tyr

85 90 95
CTT CAA ATG AAC ACC CTG AGA GCT GAG GAC AGT GCC ACT TAT TAT TGT
Leu Gln Met Asn Thr Leu Arg Ala Glu Asp Ser Ala Thr Tyr Tyr Cys

100 105 110
GCA AGA GAT AAG GGA ATA CGA TGG TAC TTC GAT GTC TGG GGC CAA GGG
Ala Arg Asp Lys Gly Ile Arg Trp Tyr Phe Asp Val Trp Gly Gln Gly

115
ACC ACG GTC ACC GTC TCC TCA
Thr Thr Val Thr Val Ser Ser

Sequenzprotokoll:
Art der Sequenz: Nukleotid mit entsprechendem Protein
Sequenzlänge: 292 Basenpaare
Strangform: Einzelstrang
Topologie: linear
Art des Moleküls: cDNA zu mRNA
Ursprünglicher Herkunftsorganismus: Maus

Merkmale:
cDNA Sequenz der Codons für die Aminosäuren 9–105 des VL Exons des κ-leichte Ketten-
Gens (VK), das für die leichte Kette des MAk kodiert und die dazugehörige Aminosäure-
sequenz. Die Aminosäuren sind nach Wu und Kabat numeriert.

VK

10 15 20
A GCA ATC CTG TCT GCA TCT CCA GGG GAG AAG GTC ACA ATG ACT TGC
Ala Ile Leu Ser Ala Ser Pro Gly Glu Lys Val Thr Met Thr Cys

25 30 35
AGC GCC AGC TCA AGT GTA AGT TAC ATG CAC TGG TAC CAG CAG AAG CCA
Arg Ala Ser Ser Ser Val Ser Tyr Met His Trp Tyr Gln Gln Lys Pro

40 45 50 55
GGA TCC TCC CCC AAA CCC TGG ATT TAT GCC ACA TCC AAC CTG GCT TCT
Gly Ser Ser Pro Lys Pro Trp Ile Tyr Ala Thr Ser Asn Leu Ala Ser

60 65 70
GGA GTC CCT GCT CGC TTC AGT GGC AGT GGG TCT GGG ACC TCT TAC TCT
Gly Val Pro Ala Arg Phe Ser Gly Ser Gly Ser Gly Thr Ser Tyr Ser

75 80 85
CTC ACA ATC ATC AGA GTG GAG GCT GAA GAT GCT GCC ACT TAT TAC TGC
Leu Thr Ile Ile Arg Val Glu Ala Glu Asp Ala Ala Thr Tyr Tyr Cys

90 95 100
CAG CAG TGG AGT AGT AAC CCG CTC ACG TTC GGT GCT GGG ACC AAG CTG
Gln Gln Trp Ser Ser Asn Pro Leu Thr Phe Gly Ala Gly Thr Lys Leu

105
GAG ATC
Glu Ile

Alternativ kann auch eine humanisierte Variante des MAk sowie funktionell vergleichbare MAk, die an Zellen des hämatopoetischen Systems binden, verwendet werden.

Bei der Humanisierung werden folgende CDR (complementarity determining regions oder hypervariable Regionen) auf ein humanes V-Gen Gerüst rekombinant transplantiert. Folgende aus der Maus V-Region stammende CDR cDNA Sequenzen werden hierzu verwendet:

VH, CDR1:
GAT TAC TAC ATG AAC
Asp Tyr Tyr Met Asn

VH, CDR2:
TTT ATT TCA AAC AAA CCT AAT GGT CAC ACA ACA GAG TAC GTA GCA TCT
Phe Ile Ser Asn Lys Pro Asn Gly His Thr Thr Glu Tyr Ser Ala Ser

GTG AAG GGT
Val Lys Gly

VH, CDR3:
GAT AAG GGA ATA CGA TGG TAC TTC GAT GTC
Asp Lys Gly Ile Arg Trp Tyr Phe Aps Val

VK, CDR1:
AGG GCC AGC TCA AGT GTA AGT TAC ATG CAC
Arg Ala Ser Ser Ser Val Ser Tyr Met His

VK, CDR2:
GCC ACA TCC AAC CTG GCT TCT
Ala Thr Ser Asn Leu Ala Ser

VK, CDR3:
CAG CAG TGG AGT AGT AAC CCG CTC ACG
Gln Gln Trp Ser Ser Asn Pro Leu Thr

## Patentansprüche

1. Radioimmunkonjugat nach Patent 198 13 687.0, bei dem die α-Strahler oder β-Strahler ohne Verwendung von Komplexbildnern stabil an den Antikörper gekoppelt sind, wobei das Radioisotop kein Jod ist.

2. Radioimmunkonjugat nach Patent 198 13 687.0, bei dem die α- oder β-Strahler stabil an den Antikörper gekoppelt sind, wobei das Radioisotop kein Jod ist,
dadurch gekennzeichnet,
dass die α- oder β-Strahler unter Verwendung von nicht immunogenen Komplexbildnern stabil an den Antikörper gekoppelt sind, wobei das Radionuklid kein Jod ist.

3. Radioimmunkonjugat nach einem der Ansprüche 1 oder 2, dadurch gekennzeichnet, dass der β-Strahler Yttrium-90, Rhenium-188, Rhenium-186, Kupfer-67 sowie Holmium-166 und Samarium-153 ist.

4. Radioimmunkonjugat nach einem der Ansprüche 1 oder 2, dadurch gekennzeichnet, dass der α-Strahler Astatin-211 oder Bismuth-212 ist.

5. Radioimmunkonjugat nach einem oder mehreren der Ansprüche 1 bis 4, dadurch gekennzeichnet, dass der MAk-Anteil des Konjugates von der Maus, dem Menschen oder anderen Säugetieren stammt, sowie intakt, fragmentiert, humanisiert oder rekombinant manipuliert ist.

6. Radioimmunkonjugat nach einem oder mehreren der Ansprüche 1 bis 4, dadurch gekennzeichnet, dass der MAk an ein extrazelluläres Antigen, welches präferentiell auf Zellen des hämatopoetischen Systems vorkommt, bindet.

7. Radioimmunkonjugat nach einem oder mehreren der Ansprüche 1 bis 4, dadurch gekennzeichnet, dass der MAk an ein Antigen auf der Oberfläche von Granulozyten oder Granulozyten-Vorläufern oder beiden Zellarten bindet.

8. Radioimmunkonjugat nach einem oder mehreren der Ansprüche 1 bis 4, dadurch gekennzeichnet, dass der MAk-Anteil des Konjugates an ein Epitop oder Epitope von CD 66 bindet.

9. Radioimmunkonjugat nach einem oder mehreren der Ansprüche 1 bis 4, dadurch gekennzeichnet, dass der MAk-Anteil des Konjugates an CD 66 a, b, c und e bindet.

10. Radioimmunkonjugat nach einem oder mehreren der Ansprüche 1 bis 4, dadurch gekennzeichnet, dass der MAk-Anteil des Konjugates der in Anlage 1 über seine V-Region definierte MAk ist oder eines seiner funktionellen Analoge, Fragmente, bzw. humanisierten gentechnischen Varianten ist.

11. Radioimmunkonjugat nach den Ansprüchen 1 bis 4, dadurch gekennzeichnet, dass die Bindung des Radionuklids an den MAk wie in den Beispielen 1 bis 10 beschrieben erfolgt.

12. Radioimmunkonjugat nach Anspruch 3, gekennzeichnet durch die therapeutische Verwendung bei hämatopoetischen Erkrankungen, aber auch von soliden Tumoren, deren Fernmetastasen sowie entzündlichen Prozessen.

13. Radioimmunkonjugat nach Anspruch 4, gekennzeichnet durch die therapeutische Verwendung bei hämatopoetischen Erkrankungen, aber auch von soliden Tumoren, deren Fernmetastasen sowie entzündlichen Prozessen.

14. Radioimmunkonjugat nach Anspruch 6, gekennzeichnet durch, die therapeutische Verwendung bei hämatopoetischen Erkrankungen, aber auch von soliden Tumoren, deren Fernmetastasen sowie entzündlichen Prozessen.

15. Radioimmunkonjugat nach Anspruch 7, gekennzeichnet durch, die therapeutische Verwendung von hämatopoetischen Erkrankungen, aber auch von soliden Tumoren, deren Fernmetastasen sowie entzündlichen Prozessen.

16. Verwendung des Radioimmunkonjugat nach einem oder mehreren der Ansprüche 1 bis 8, 9 oder 10, dadurch gekennzeichnet, dass es mit oder ohne hoch dosierte Chemotherapie und/oder Ganzkörperbestrahlungen bei hämatopoetischen Erkrankungen anwendbar ist.

17. Radioimmunkonjugat, gekennzeichnet durch Erzeugung einer Radioaktivitätsverteilung, bei der > 10 % der injizierten Dosis im Knochenmark lokalisiert.

18. Radioimmunkonjugat nach einem der Ansprüche 1 bis 4, gekennzeichnet durch Erzeugung einer Radioaktivitätsverteilung im Menschen gemäss Beispiel 9.

19. Radioimmunkonjugat nach einem oder mehreren der Ansprüche 1 bis 10, dadurch gekennzeichnet, dass es in Verbindung mit Chemotherapie und/oder Ganzkörperbestrahlung im Rahmen einer Therapie hämatopoetischer Erkrankungen, aber auch von Tumoren und Metastasen anwendbar ist.

20. Radioimmunkonjugat nach einem oder mehreren der Ansprüche 1 bis 10 gekennzeichnet zur Verwendung zur Mono-Therapie von akuten und chronischen myeloischen Leukämien.

21. Radioimmunkonjugat nach einem oder mehreren der Ansprüche 1 bis 10, gekennzeichnet zur Verwendung bei der Behandlung von akuten und chronischen lymphatischen Leukämien und Lymphomen in Kombination mit gezielter Chemotherapie oder Ganzkörperbestrahlung.

22. Radioimmunkonjugat nach einem oder mehreren der Ansprüche 1 bis 10, gekennzeichnet durch die therapeutische Verwendbarkeit in Verbindung mit literaturbekannten Liganden mit Spezifität für lymphozytäre Antigene wie CD 19, CD 20, CD 22, HLL 2 und HLA DR 10β.

23. Radioimmunkonjugat nach einem oder mehreren der Ansprüche 1 bis 10, gekennzeichnet durch ihre therapeutische Verwendbarkeit als bispezifische Antikörperradioimmunkonjugate nach Beispiel 14 mit Spezifität gegen granoluzytäre Antigene, lymphozytäre Antigene oder Antigene auf Knochenmark Stammzelle.

24. Radioimmunkonjugat nach einem oder mehreren der Ansprüche 1 bis 10, sowie Ansprüchen 22 und 23, gekennzeichnet durch ihre therapeutische Verwendbarkeit als bispezifische Antikörperradioimmunkonjugate nach Beispiel 14, mit Spezifität entsprechend Anlage 1, kombiniert mit Spezifitäten wie anti CD 19, anti CD 20, anti CD 22, anti HLA DR 10β oder HLL2.

25. Radioimmunkonjugat nach einem oder mehreren der Ansprüche 1 bis 10, sowie den Ansprüchen 22 und 23, gekennzeichnet durch therapeutische Verwendbarkeit in Verbindung mit einer nachfolgenden Anwendung von Komplexbildnern in Form von löslichen Salzen.

26. Radioimmunkonjugat nach einem oder mehreren der Ansprüche 1 bis 10, gekennzeichnet durch therapeutische Verwendbarkeit als Zweiphasen-Radioimmunkonjugat, bestehend aus einem biotinilierten MAk und mit α- oder β-Strahlern markierten Avidin zur Therapie von malignen hämopoetischen Erkrankungen.

27. Radioimmunkonjugat nach einem oder mehreren der Ansprüche 1 bis 10, gekennzeichnet durch therapeutische Anwendbarkeit als Zweiphasen-Radioimmunkonjugat, bestehend aus einem biotinilierten MAk mit α- oder β-Strahlern markierten aviden Bindemoleküle wie Straptavidin oder deren rekombinant, biochemisch oder peptidchemisch herstellbaren Fragmente zur Therapie von malignen hämopoetischen Erkrankungen.

28. Verwendung von Radioimmunkonjugaten entsprechend den Ansprüchen 1 bis 10 zur Behandlung des malignen Pemphigus.

29. Verwendung von Radioimmunkonjugaten entsprechend den Ansprüchen 1 bis 10 zur Behandlung des malignen Pemphigus in Kombination mit Knochenmarktransplantationen.

30. Verwendung von Radioimmunkonjugaten entsprechend den Ansprüchen 1 bis 10 zur Behandlung des Mammakarzinoms, vorzugsweise dessen Knochenmarksmetastasen.

31. Verwendung von Radioimmunkonjugaten entsprechend den Ansprüchen 1 bis 10 zur Behandlung des Prostatakarzinoms, vorzugsweise dessen Knochenmarksmetastasen.

32. Verwendung von Radioimmunkonjugaten entsprechend den Ansprüchen 1 bis 10 zur Behandlung von Lymphomen, vorzugsweise deren Knochenmarksmetastasen.

33. Verwendung von Radioimmunkonjugaten entsprechend den Ansprüchen 1 bis 10 zur Behandlung von Knochenmarksmetastasen aller Tumore die ins Knochenmark metastasieren können wie z. B. Lungenkarzinome, Mammakarzinome, Prostatakarzinome, Pankreaskarzinome, Magenkarzinome, Leberkarzinome, Nierenkarzinome, Colonkarzinome, Rektumkarzinome, Hodentumore, Ovarialkarzinome, Melanome, Lymphome, Mesotheliome, Kaposisarkome, Hämangiome, Sarkome, Osteosarkome, Blasenkarzinome und Hals-, Nasen-, Ohren-Tumore.

34. Verwendung von Radioimmunkonjugaten entsprechend den Ansprüchen 1 bis 10 zur Knochenmarksdepletion vor einer Transplantation.

35. Verwendung von Radioimmunkonjugaten als Monotherapie oder in Verbindung mit Chemotherapie und/oder Ganzkörperbestrahlung zur Therapie von chronisch myeloischen Leukämien, akuten myeloischen Leukämien sowie Knochenmarksmetastasen von Lungenkarzinomen, Mammakarzinomen, Prostatakarzinomen, Pankreaskarzinomen, Magenkarzinomen, Leberkarzinomen, Nierenkarzinomen, Colonkarzinomen, Rektumkarzinomen, Hodentumoren, Ovarialkarzinomen, Melanomen, Lymphomen, Mesotheliomen, Kaposisarkomen, Hämangiomen, Sarkomen, Osteokarzinomen, Blasenkarzinomen und Hals-, Nieren-, Ohren-Tumoren.

36. Verwendung von mehr als einem Radioimmunkonjugat zur Therapie von Erkrankungen bei denen eine Knochenmarkskonditionierung durchgeführt wird.
